# EUROPEAN PATENT APPLICATION

(11) **EP 4 529 930 A1**
(43) Date of publication of application: **02.04.2025**
(21) Application number: 22950073.1
(22) Date of filing: 02.12.2022
(51) Int. Cl.: A61L 2/08, A61L 2/20, A61B 5/145, A61B 5/1486

(54) **METHOD FOR STERILIZING MEDICAL INSTRUMENT KIT**

(30) Priority: 05.07.2022 CN 202210785041
(71) Applicant: Shenzhen Sisensing Co., Ltd., Shenzhen, Guangdong 518110 (CN)
(72) Inventor: CHEN, Zhi, Shenzhen, Guangdong 518110 (CN); XIA, Bin, Shenzhen, Guangdong 518110 (CN); LI, Yunfeng, Shenzhen, Guangdong 518110 (CN)
(74) Representative: Isern Patentes y Marcas S.L.
(86) International application number: PCT/CN2022/136107
(87) International publication number: WO 2024/007516

(57) **Abstract**

The present disclosure provides a sterilization method for a medical instrument suite (1000), the medical instrument suite (1000) including an analyte sensor (400), an electronic device (300), a lower cartridge (200) having an accommodating chamber (210), and an upper cartridge (100) sealingly assembled with the lower cartridge (200), wherein the analyte sensor (400) has a sensing portion (410) that may react with an analyte to generate a sensing signal, and a connecting portion (420) electrically connected to the sensing portion (410); the electronic device (300) is configured to be able to couple with the connecting portion (420) and receive the sensing signal generated by the sensing portion (410), the sterilization method includes placing the sensing portion (410) of the analyte sensor (400) in the accommodating chamber (210) of the lower cartridge (200), and the accommodating chamber (210) is sealed in such a manner that the sensing portion (410) is sealed; the lower cartridge (200) is irradiated with an electron beam, the electronic device (300) is coupled to the connecting portion (420), and the upper cartridge (100) and the lower cartridge (200) are sealingly assembled. The sterilization method involved in the present disclosure is simple and convenient in sterilization operation and may facilitate the subsequent use of the medical instrument suite (1000) by a user.

## Description

### Background of invention

### Field of Invention

The present disclosure generally relates to the biomedical engineering industry, and in particular to a sterilization method for a medical instrument suite.

### Description of Related Art

For clinical diagnosis or personal health monitoring, it is often necessary to monitor various analytes in the human body by using an analyte sensor. For example, for patients with diabetes, it is necessary to monitor the glucose concentration of the interstitial fluid in real-time and continuously at ordinary times, and to adjust the glucose concentration in time, for example, by adjusting diet or applying the medication, etc. so as to reduce the possibility of complications due to abnormal glucose concentration.

Currently, generally users monitor the glucose concentration of the interstitial fluid by using a glucose sensor capable of being inserted under the skin and reacting with the glucose in the interstitial fluid and an electronic device applied to the skin and connected to the glucose sensor. In order to insert the glucose sensor beneath the skin and to apply the electronic device to the skin, it is often necessary to resort to an insertion device. Specifically, the insertion device includes a needle capable of being pierced into the skin to inserting the glucose sensor via the needle beneath the skin, and then separating the needle from the glucose sensor and removing the needle from the skin.

For consideration of user's health and safety, the glucose sensor and needle implanted into the human body or positioned under the skin should be sterile when inserted. Sterilization is therefore often required to effectively eliminate or kill transmissible factors (such as bacteria, fungi, and viruses). A commonly used sterilization method, such as electron beam sterilization, may be used to terminally sterilize the sensor, however, electron beam sterilization may cause damage to the electronic device and affect the normal function thereof. A commonly used sterilization method, such as ethylene oxide sterilization, may sterilize electronic components via gaseous chemical sterilization, however, ethylene oxide may affect the activity of chemicals in a sensor. In the prior art, the Chinese patent with the publication No. CN112423664A entitled "Focused Sterilization and Sterilized SubAssemblies for Analyte Monitoring Systems" discloses a sterilization method. The patent uses a mode of mutual shielding to protect two kinds of components so as to achieve the purpose of sterilization; when sterilizing the electronic portion, a mode of liquid plus sealing is used to protect the sensor portion from being affected by a chemical gas; when sterilizing the sensor portion, a mode of using a shielding material in the outer housing portion of the electronic device is used, so that the electron beam does not affect the electronic portion; however, this sterilization mode requires an additional shielding structure and is relatively complicated. It is also common in the prior art to sterilize the sensor and the electronic device separately and then package them separately; however, such sterilization mode requires additional assembly when used by a user, resulting in inconvenience in use.

### Summary of invention

The present disclosure is completed in view of the state of the prior art mentioned above; an object of the present disclosure is to provide a sterilization method for a medical instrument suite; according to the sterilization method of the present disclosure, the sterilization operation is simple and convenient, subsequent use by a user can be facilitated.

For the above purpose, the present disclosure provides a sterilization method for a medical instrument suite, the medical instrument suite including an analyte sensor, an electronic device, a lower cartridge having an accommodating chamber, and an upper cartridge sealingly assembled with the lower cartridge, wherein the analyte sensor has a sensing portion capable of reacting with an analyte to generate a sensing signal, and a connecting portion electrically connected to the sensing portion; the electronic device is configured to be capable of coupling with the connecting portion and receiving the sensing signal generated by the sensing portion, the sterilization method includes placing the sensing portion of the analyte sensor in the accommodating chamber of the lower cartridge, sealing the accommodating chamber in such a manner that the sensing portion is sealed; irradiating the lower cartridge with an electron beam, coupling the electronic device to the connecting portion, and sealingly assembling the upper cartridge and the lower cartridge.

In the present disclosure, the sensing portion of the analyte sensor is placed in a sealed accommodating chamber; after the sensing portion in the accommodating chamber is sterilized by electron beam irradiation, the electronic device and the sensor are assembled; finally, the upper cartridge and the lower cartridge are assembled. In this case, the electronic beam can be used to sterilize the sensing portion of the sensor; at the same time, the electronic device can be prevented from being affected by the electronic beam; finally, the upper cartridge, electronic device, analyze sensor, and lower cartridge are assembled such that the user does not need to perform additional assembly when using it, facilitating the subsequent use by the user.

In addition, in the sterilization method according to the present disclosure, optionally, the irradiation dose of the electron beam is greater than or equal to 15kGy and less than or equal to 50kGy. Therefore, a target component is sterilized with an appropriate irradiation dose of the electron beam to enable microorganisms such as bacteria in the target component to be effectively killed without adversely affecting the material of the component of the medical instrument suite by the electron beam.

In addition, in the sterilization method to which the present disclosure relates, optionally, the medical instrument suite further includes a puncturing member, wherein the puncturing member includes a main body portion, and a sharp object provided on the main body portion and having an accommodating groove for accommodating the sensing portion, the sharp object being sealed in the accommodating chamber. In this case, the sharp object of the puncturing member can be packaged in the accommodating chamber together with the sensing portion of the sensor, whereby the sharp object and the sensing portion can be sterilized simultaneously using the electron beam and are not affected by microorganisms in the environment outside the accommodating chamber after the sterilization.

In addition, in the sterilization method to which the present disclosure relates, optionally, a projection is performed along a radial direction of the instrument suite and the puncturing member is covered by the upper cartridge. In this case, the puncturing member is not exposed so that the user can be prevented from being mistakenly injured by the puncturing member as much as possible.

In addition, optionally, the sterilization method to which the present disclosure relates further includes placing the electronic device in a chemical gas atmosphere after coupling the electronic device to the connecting portion. Therefore, the electronic device can be sterilized by using a chemical gas.

In addition, in the sterilization method to which the present disclosure relates, optionally, the chemical gas is ethylene oxide. Therefore, the electronic device can be sterilized by using ethylene oxide.

In addition, in the sterilization method to which the present disclosure relates, optionally, the accommodating chamber is sealed with wax in a molten state. Therefore, the accommodating chamber can be sealed with a wax seal, and when the analyte sensor needs to be used, the wax seal can be broken by mechanical force (pulling out the analyte sensor) while minimizing the damage to the analyte sensor.

In addition, in the sterilization method to which the present disclosure relates, optionally, the electronic device is not within an irradiation range of the electron beam. Therefore, the electron beam can be prevented from affecting the electronic device.

In addition, in the sterilization method to which the present disclosure relates, optionally, the lower cartridge further includes a desiccant provided in the accommodating chamber. Thereby, the environment inside the accommodating chamber can be brought into a dry state by the desiccant, so that the sensing portion and/or the sharp object sealed in the accommodating chamber is not affected by the moisture in the environment.

In addition, in the sterilization method to which the present disclosure relates, optionally, an outer contour of the upper cartridge is matched with an inner contour of the lower cartridge, and an outer edge of the upper cartridge includes a rubber ring. Thereby, the upper cartridge and the lower cartridge can be assembled, and the sealability after the assembly can be increased by the rubber ring.

According to the present disclosure, a sterilization method for a medical instrument suite can be provided; according to the sterilization method for the present disclosure, the sterilization operation is simple and convenient, and the subsequent use by a user can be facilitated.

### Brief description of the drawings

Fig. 1 is an application diagram showing a medical instrument suite to which the present disclosure relates.
Fig. 2A is a schematic diagram showing an analyte sensor and an electronic device to which the present disclosure relates.
Fig. 2B is a schematic structural diagram showing an assembled analyte sensor, electronic device, and puncturing member to which the present disclosure relates.
Fig. 2C is an exploded diagram showing an analyte sensor, an electronic device, and a puncturing member to which the present disclosure relates.
Fig. 2D is a bottom diagram showing a loading stage to which the present disclosure relates.
Fig. 2E is a schematic structural diagram showing an analyte sensor to which the present disclosure relates.
Fig. 2F is a schematic structural diagram showing a sharp object to which the present disclosure relates.
Fig. 3 is an overall flow chart showing a sterilization method for a medical instrument suite to which the present disclosure relates.
Fig. 4 is a schematic diagram showing an upper cartridge and a lower cartridge before and after the assembly to which the present disclosure relates.
Fig. 5A is a rear diagram showing a lower cartridge to which the present disclosure relates.
Fig. 5B is a cross-sectional diagram showing the lower cartridge of Fig. 4A in the direction of BB' according to the present disclosure.
Fig. 5C shows an enlarged partial diagram of Fig. 4B according to the present disclosure.
Fig. 6A is a schematic diagram showing an upper cartridge after the picking up to which the present disclosure relates.
Fig. 6B shows a cross-sectional diagram of an upper cartridge after the picking up to which the present disclosure relates.
Fig. 7 is a flow chart showing a sterilization method for a medical instrument suite to which the present disclosure relates.

### Detailed description

Hereinafter, the present disclosure will be described in further detail with reference to the accompanying drawings and specific implementation modes. In the drawings, the same reference numerals are used for the same parts or parts having the same function, and a repeated description thereof will be omitted.

The implementation modes of the present disclosure relate to a sterilization method for a medical instrument suite. Hereinafter, a sterilization method for a medical instrument suite according to the specific implementation mode of the present disclosure will be described in detail with reference to the accompanying drawings.

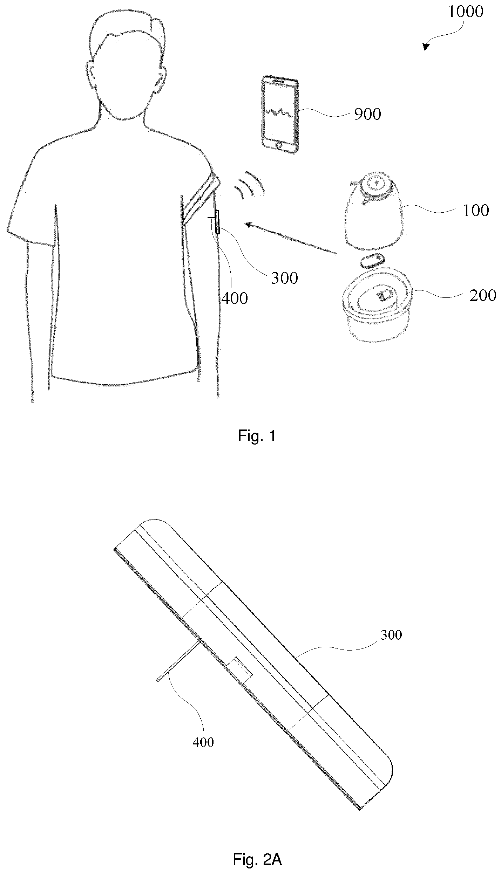

Fig. 1 is an application diagram showing a medical instrument suite 1000 to which the present disclosure relates.

As shown in Fig. 1, in some examples, the medical instrument suite 1000 may include an analyte sensor 400 that may acquire physiological information of a host, an upper cartridge 100 that may apply the analyte sensor 400 to the host, an electronic device 300 that may receive the physiological information acquired by the analyte sensor 400, and a lower cartridge 200 that may house the analyte sensor 400.

In some examples, analyte sensor 400 may generate information of a particular analyte in a body fluid based on the body fluid, etc., for example, reacting with the analyte in the body fluid and generating analyte information. In this case, the sensor reacts with the analyte in the body fluid, whereby the acquisition of the analyte information in the body fluid can be facilitated.

In some examples, analyte sensor 400 and electronic device 300 may be applied to the host together. Thereby, the analyte sensor 400 can acquire the physiological information of the host and transfer the acquired physiological information to the electronic device 300.

Although Fig. 1 illustrates the wearing positions of the analyte sensor 400 and the electronic device 300, the present embodiment is not limited to this, for example, the analyte sensor 400 and electronic device 300 may also be worn on the abdomen, waist, legs, etc.

In addition, the present disclosure also provides a monitoring system, the monitoring system may include an analyte sensor 400 that may acquire physiological information of a host, an electronic device 300 that may receive the physiological information acquired by the analyte sensor 400, and a reading device 900 that may be communicatively connected to the electronic device 300 (see Fig. 1) according to the present embodiment. The analyte sensor 400 applied to the host may transmit the acquired physiological information to the reading device 900, for example, wirelessly through the electronic device 300, thereby facilitating the host to read and monitor the physiological information thereof.

In the present embodiment, the analyte targeted by the analyte sensor 400 device may be one or more of glucose, uric acid, myocardial enzyme, lactate, dopamine, acetylcholine, amylase, bilirubin, cholesterol, chorionic gonadotropin, creatine kinase, creatine, creatinine, DNA, fructosamine, glutamine, growth hormone, hormones, ketone body, lactate, oxygen, peroxide, prostate specific antigen, prothrombin, RNA, thyroid stimulating hormone, or troponin.

Hereinafter, the medical instrument suite 1000 according to an example of the present embodiment will be described with an example mainly with glucose as an analyte. It needs to be noted that for other analytes, those skilled in the art will be able to analyze other analytes with minor modifications to the adoption of the analyte sensor 400 based on glucose.

### (The electronic device 300 and the analyte sensor 400)

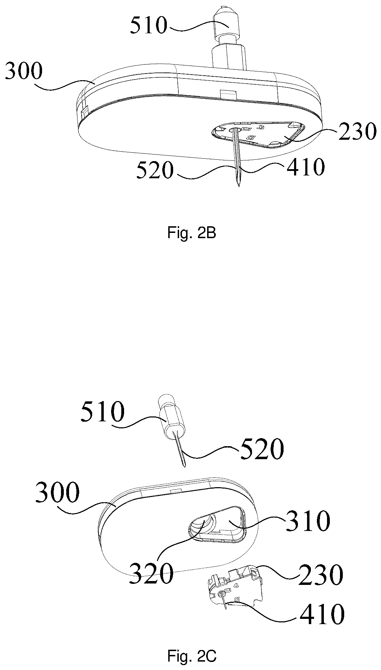

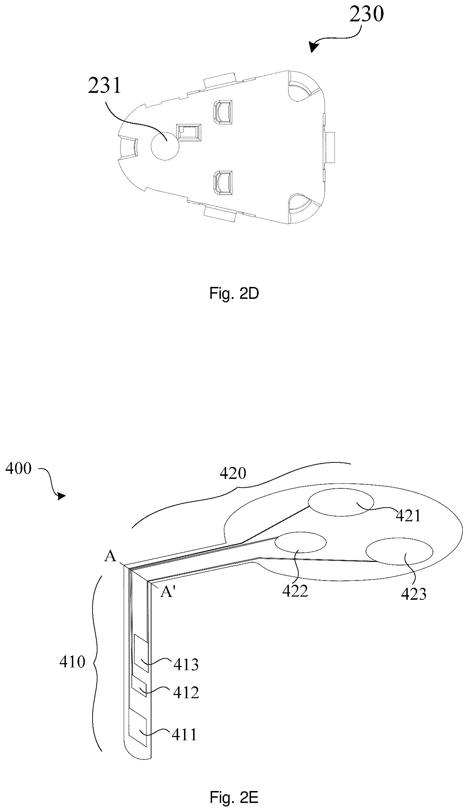

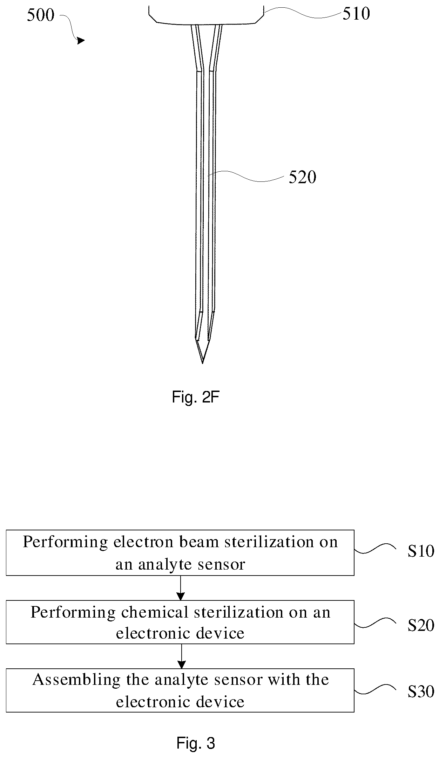

Fig. 2A is a schematic diagram showing an analyte sensor 400 and an electronic device 300 to which the present disclosure relates. Fig. 2B is a schematic structural diagram showing an assembled analyte sensor 400, electronic device 300, and puncturing member 500 to which the present disclosure relates. Fig. 2C is an exploded diagram showing an analyte sensor 400, an electronic device 300, and a puncturing member 500 to which the present disclosure relates. Fig. 2D is a bottom diagram showing a loading stage 230 to which the present disclosure relates. Fig. 2E is a schematic structural diagram showing an analyte sensor 400 to which the present disclosure relates. Fig. 2F is a schematic structural diagram showing a sharp object 520 to which the present disclosure relates.

As described above, in the present embodiment, the medical instrument suite 1000 may include an electronic device 300 and an analyte sensor 400 (see Fig. 2A).

In some examples, referring to Fig. 1 and Fig. 2A, the electronic device 300 and the analyte sensor 400 may ultimately be applied to a host together. Wherein, the analyte sensor 400 may be partially implanted into a host, and the electronic device 300 may be connected to the analyte sensor 400 and applied to the body surface of the host.

In some examples, the analyte sensor 400 may acquire physiological information of a host. The electronic device 300 may receive physiological information of an analyte generated by the analyte sensor 400. In some examples, the electronic device 300 may further process the physiological information of the analyte. In some examples, the electronic device 300 may send the physiological information of the analyte or the processed physiological information of the analyte to other devices.

In some examples, the surface of the electronic device 300 proximate to and connected to the analyte sensor 400 may be an adhesive surface. Thereby, the electronic device 300 can be attached to the skin surface by adhesive attraction.

Generally, for the consideration of the health and safety of the host, the part of analyte sensor 400 implanted into the body needs to be sterilized, the common sterilization methods include electron beam sterilization and ethylene oxide sterilization. However, the analyte sensor 400 usually has chemical substances (e.g., an enzyme), and the activity of chemical substances will be affected by ethylene oxide; the electron beam sterilization will damage the function of the electronic device 300. Therefore, the analyte sensor 400 and the electronic device 300 can not be sterilized simultaneously.

### (Analyte sensor 400)

In some examples, referring to Fig. 2E, the analyte sensor 400 may include a sensing portion 410 and a connecting portion 420. In some examples, the sensing portion 410 of the analyte sensor 400 may be implanted in a body surface, such as a human body, to be in contact with interstitial fluid in the body. In some examples, the connecting portion 420 may be connected to an electronic device 300 located on the body surface. In the operation of the analyte sensor 400, the sensing portion 410 may react with the interstitial fluid in the body to generate a sensing signal (e.g., a current signal) and transmit the sensing signal through the connecting portion 420 to the electronic device 300 on the body surface, and the electronic device 300 processes the sensing signal to obtain the concentration of the analyte.

In the present embodiment, although the analyte sensor 400 directly inspects the analyte in the interstitial fluid, the concentration of the analyte in the interstitial fluid tends to have a strong correlation with the concentration of the analyte in the blood, and the concentration of the analyte in the blood may also be determined through inspecting the analyte in the interstitial fluid.

In some examples, the length of sensing portion 410 may be determined according to the depth of the implantation under the skin, and the depth of the implantation under the skin may be determined according to the location to be pierced. In some examples, the width of sensing portion 410 may be limited to about 0.5mm or less. Generally, the narrower the width of the sensing portion 410 is, the less painful the host feels during the implantation and during the use.

In some examples, the sensing portion 410 may include a working electrode 411, a reference electrode 412, and a counter electrode 413, and the connecting portion 420 may include a first contact 421 connected with the working electrode 411 via a lead, a second contact 422 connected with the reference electrode 412 via a lead, and a third contact 423 connected with the counter electrode 413 via a lead.

In some examples, the working electrode 411 may have a sensing reagent that may include an enzyme and a redox mediator. Taking glucose as an analyte as an example, the enzyme may be glucose oxidase or glucose dehydrogenase. The redox mediators (ROMs) may accelerate the transfer of electrons between an electron donor and an electron acceptor by cyclically switching the oxidation state and reduction state of the redox mediators. Thereby, the glucose in the body fluid of a human body can undergo a redox reaction under the catalysis of an enzyme and form the transfer of electrons, resulting in converting the concentration signal of the glucose in tissues into an electrical signal.

In some examples, the reference electrode 412 may form a known and fixed potential difference with the interstitial fluid or the blood. In this case, the potential difference between the working electrode 411, and the interstitial fluid or the blood may be measured by the potential difference formed between the reference electrode 412 and the working electrode 411, so that the voltage generated by the working electrode 411 may be accurately mastered; thereby, the electronic device 300 can automatically adjust and maintain the stability of the voltage at the working electrode 411 according to a preset voltage value, so that the measured current signal can more accurately reflect the glucose concentration value.

In some examples, the counter electrode 413 may form a loop with the working electrode 411 to generate a current signal.

In addition, in the present embodiment, the working electrode 411, the reference electrode 412, and the counter electrode 413 of the sensing portion 410 are arranged in a distributed manner, but the embodiment of the present disclosure is not limited thereto and may include a side-by-side (in parallel) arrangement.

In some examples, the current signal generated by sensing portion 410 may be transmitted to a contact (e.g., the first contact 421, the second contact 422, or the third contact 423) of the connecting portion 420 via a lead. In some examples, the contact (e.g., the first contact 421, the second contact 422, or the third contact 423) may be connected to the electronic device 300, whereby a physiological signal obtained by the sensing portion 410 can be transmitted to the electronic device 300 for analysis via the connecting portion 420. The electronic device 300 thus analyzes and obtains the concentration of the analyte.

It needs to be noted that Fig. 2E only schematically shows the shape of the analyte sensor 400, and the present embodiment is not limited thereto, and the analyte sensor 400 may have other shapes.

### (Puncturing member 500)

In this embodiment, in some examples, the medical instrument suite 1000 may include a puncturing member 500 (see Fig. 2F).

In some examples, the puncturing member 500 may be configured to partially implant the analyte sensor 400 into a host. In some examples, the puncturing member 500 may implant the sensing portion 410 of the analyte sensor 400 into a host. In some examples, the puncturing member 500 implants the sensing portion 410 into the host by simultaneously puncturing the user. In some examples, for the consideration of the health and safety of the host, the portion of the puncturing member 500 that needs to pierce into the user may also needs to be sterilized.

In some examples, the puncturing member 500 may include a main body portion 510 and a sharp object 520. In some examples, the sharp object 520 may be provided on the main body portion 510. In some examples, the sharp object 520 may be sterilized.

In some examples, referring to Fig. 2F, the sharp object 520 may have an accommodating groove. In some examples, the accommodating groove of the sharp object 520 may accommodate the sensing portion 410.

In some examples, the sharp object 520 may be made of stainless steel. In this case, the risk of using the sharp object 520 can be reduced, and the sharp object 520 made of stainless steel has sufficient hardness to easily puncture the skin and facilitate the use of an object to be inspected. Additionally, in some examples, the sharp object 520 may also be made of plastics, glasses, or metals. Thereby, the manufacturing cost of the sharp object 520 can be controlled.

(The assembly of the electronic device 300, the analyte sensor 400, and the puncturing member 500)

As shown in Fig. 2B, Fig. 2C, and Fig. 2D, the medical instrument suite 1000 may include a loading stage 230.

In some examples, the loading stage 230 may be used to load the analyte sensor 400. In some examples, the loading stage 230 may be used to load the connecting portion 420. In some examples, the top-view profile of the loading stage 230 and the top-view profile of the connecting portion 420 may be substantially the same, thereby facilitating the loading of the connecting portion 420.

In some examples, the loading stage 230 may have a hollow portion 231, and in some examples, the sensing portion 410 may pass through the hollow portion 231. In some examples, when the connecting portion 420 is loaded on the loading stage 230, the sensing portion 410 passes through the hollow portion 231, and at this time, the connecting portion 420 and the sensing portion 410 are perpendicular to each other.

In some examples, the sharp object 520 of the puncturing member 500 may penetrate into the hollow portion 231, enabling the sensing portion 410 to be accommodated in an accommodating groove of the sharp object 520.

In some examples, the electronic device 300 may include a receiving portion 310. In some examples, the receiving portion 310 may be formed as a shape to match the loading stage 230, and the loading stage 230 may be provided in the receiving portion 310 in embedding manner. In some examples, the electronic device 300 and the connecting portion 420 may be coupled when the loading stage 230 on which the connecting portion 420 is loaded is provided in the receiving portion 310 in embedding manner.

In some examples, the electronic device 300 may include a through-hole 320. In some examples, sequentially the connecting portion 420 may be enabled to be loaded on the loading stage 230 and the sensing portion 410 may be enabled to penetrate out of the hollow portion 231, the sharp object 520 of the puncturing member 500 may be enabled to penetrate into the hollow portion 231, and the receiving portion 310 of the electronic device 300 may be enabled to be fitted (assembled) with the loading stage 230, at which time the main body portion 510 of the puncturing member 500 may pass through the through-hole 320 of the electronic device 300. Thereby, the sequential assembly of the analyte sensor 400, the puncturing member 500, and the electronic device 300 can be achieved.

In some examples, after sensing portion 410 and sharp object 520 penetrate out of the hollow portion 231, the sensing portion 410 and the sharp object 520 are positioned within the accommodating chamber 210 (described in detail later).

Fig. 3 is an overall flow chart showing a sterilization method for a medical instrument suite 1000 to which the present disclosure relates.

In some examples, the sterilization method for the medical instrument suite 1000 may include: performing electron beam sterilization on an analyte sensor 400 (step S10), performing chemical sterilization on an electronic device 300 (step S20), and assembling the analyte sensor 400 with the electronic device 300 (step S30). In this case, by separately sterilizing the analyte sensor 400 and the electronic device 300 using different sterilization methods, it is able to improve the safety of the use of the host without causing the electron beam sterilization to affect the function of the electronic device 300 and without affecting the function of analyte sensor 400 through chemical sterilization, so that the sterilized analyte sensor 400 and electronic device 300 may be easily applied to a host after being assembled.

In some examples, the sterilization method for the medical instrument suite 1000 may further include performing electron beam sterilization or chemical sterilization on the puncturing member 500.

In some examples, in step S30, the analyte sensor 400, the puncturing member 500, and the electronic device 300 may further be assembled.

### (The assembly of the upper cartridge 100 and the lower cartridge 200)

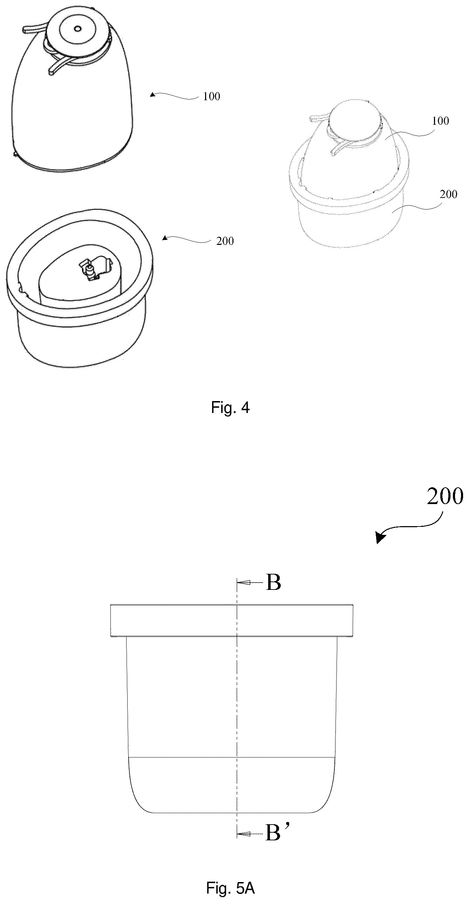

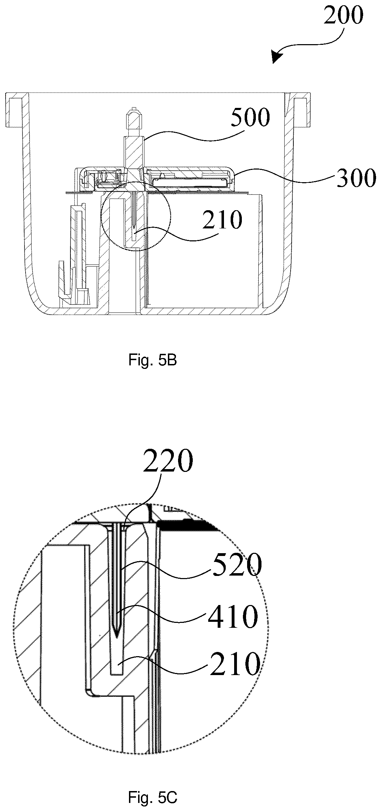

Fig. 4 is a schematic diagram showing an upper cartridge 100 and a lower cartridge 200 before and after the assembly to which the present disclosure relates. Fig. 5A is a rear diagram showing a lower cartridge 200 to which the present disclosure relates. Fig. 5B is a cross-sectional diagram showing the lower cartridge 200 in the direction of BB' in Fig. 4A according to the present disclosure. Fig. 5C shows an enlarged partial diagram of Fig. 4B according to the present disclosure.

As described above, in the present embodiment, the medical instrument suite 1000 may include an upper cartridge 100 and a lower cartridge 200.

In some examples, the upper cartridge 100 may be sealingly assembled with the lower cartridge 200. Thereby, the lower cartridge 200 and the upper cartridge 100 may be kept in a sealed state inside to reduce the influence of the external environment on a device inside the medical instrument suite 1000.

In some examples, as shown in Fig. 4, the upper cartridge 100 may be of a cylindrical structure having one opened end, and the lower cartridge 200 may be of a cylindrical structure having one opened end. In some examples, the outer contour of the opening of the upper cassette 100 may match the inner contour of the opening of the lower cassette 200. In this case, the sealed assembly of the upper cartridge 100 and the lower cartridge 200 can be achieved by moving the opening of the upper cartridge 100 toward the opening of the lower cartridge 200 so as to nest the upper cartridge 100 into the lower cartridge 200. Of course, in some examples, it is also possible that the inner contour of the opening of the upper cartridge 100 may be matched with the outer contour of the opening of the lower cartridge 200, and the sealed assembly of the upper cartridge 100 and the lower cartridge 200 is achieved by nesting the lower cartridge 200 into the upper cartridge 100. In other examples, the sealing assembly of the upper cartridge 100 and the lower cartridge 200 may be accomplished without nesting, for example, by spiral connection.

In some examples, the outer edge of the opening of the upper cartridge 100 may have a rubber ring. Thereby, it is able to improve the gas tightness after the upper cartridge 100 and the lower cartridge 200 are assembled.

Referring to Fig. 5A, Fig. 5B, and Fig. 5C, in some examples, the lower cartridge 200 may have an accommodating chamber 210.

In some examples, the analyte sensor 400 may be wholly or partially accommodated within the lower cartridge 200. In some examples, the sensing portion 410 of the analyte sensor 400 may be placed in the accommodating chamber 210, and accommodating chamber 210 is sealed. In this case, it is able to insulate the sensing portion 410 from the external gas, and thus it is able to reduce the contamination of the sensing portion 410 due to bacteria or the like in the external gas environment.

In other examples, the puncturing member 500 may be wholly or partially accommodated in the lower cartridge 200. In some examples, the sharp object 520 of the puncturing member 500 may be placed in the accommodating chamber 210, and the accommodating chamber 210 is sealed. In this case, the sharp object 520 can be isolated from the external gas, and the contamination of the sharp object 520 due to bacteria or the like in the external gas environment can be reduced.

In some examples, the sensing portion 410 and/or the sharp object 520 may be placed in the accommodating chamber 210 and the accommodating chamber 210 is sealed via a seal 220.

In some examples, the seal 220 may be wax in a molten state. Thereby, the sealing of the accommodating chamber 210 can be achieved, and the seal 220 can be subsequently destroyed by mechanical force. For example, when the medical instrument suite 1000 is in use, pulling out the analyte sensor 400 and/or the sharp object 520 may destroy the seal 220 so that the analyte sensor 400 and/or the sharp object 520 are pulled out of the accommodating chamber 210.

In some examples, sequentially, the analyte sensor 400 may be enabled to be loaded on the loading stage 230, the sensing portion 410 and the sharp object 520 may be enabled to penetrate out of the hollow portion 231 and be placed in the accommodating chamber 210, the accommodating chamber 210 may be enabled to be sealed, the electronic device 300 may be enabled to be assembled with the loading stage 230, and finally, the upper cartridge 100 may be enabled to be nested into the lower cartridge 200 such that the upper cartridge 100 and the lower cartridge 200 are sealed and assembled. In some examples, after the upper cartridge 100 and the lower cartridge 200 are sealingly assembled, the electronic device 300, the analyte sensor 400, and the puncturing member 500 are accommodated in the upper cartridge 100 (described later). Thereby, the medical instrument suite 1000 according to the present embodiment is obtained.

### (Picking up the electronic device 300 and the analyte sensor 400)

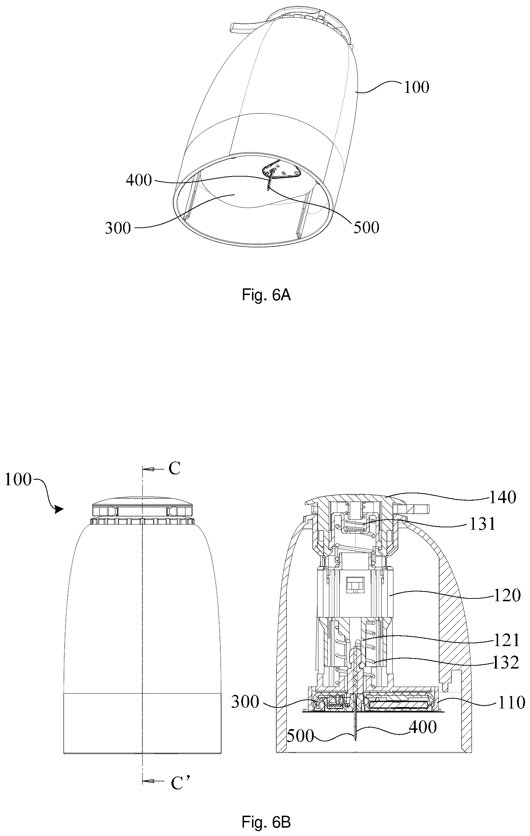

Fig. 6A is a schematic diagram showing an upper cartridge 100 after being picked up to which the present disclosure relates. Fig. 6B shows a cross-sectional diagram of an upper cartridge 100 after being picked up to which the present disclosure relates. Fig. 6A is a side diagram of the upper cartridge 100, and Fig. 6B is a cross-sectional diagram of Fig. 6A in the direction of CC'.

In this embodiment, the upper cartridge 100 may also be generally referred to as a sensor application device, a needle aid, a needle aiding device, etc. In some examples, when the analyte sensor 400 is needed, the upper cartridge 100 may pick up and apply the analyte sensor 400 to a host. In some examples, the upper cartridge 100 may be configured to pick up the electronic device 300 and apply the electronic device 300 to a body surface of a host. In some examples, the upper cartridge 100 may be configured to pick up the puncturing member 500 and pierce the puncturing member 500 into the skin of a subject such that the analyte sensor 400 pierces into the skin and then the puncturing member 500 is pulled out (rebounds).

In some examples, as mentioned above, sequentially, the analyte sensor 400 may be enabled to be loaded on the loading stage 230 of the lower cartridge 200, the sensing portion 410 and the sharp object 520 may be enabled to penetrate out of the hollow portion 231 and be placed in the accommodating chamber 210, the accommodating chamber 210 may be enabled to be sealed, the electronic device 300 may be enabled to be assembled with the loading stage 230, and finally, the upper cartridge 100 may be enabled to be nested into the lower cartridge 200 such that the upper cartridge 100 and the lower cartridge 200 are sealed and assembled. In some examples, after the upper cartridge 100 and the lower cartridge 200 are sealingly assembled, the upper cartridge 100 picks up the electronic device 300, the analyte sensor 400, and the puncturing member 500 such that the electronic device 300, the analyte sensor 400, and the puncturing member 500 are accommodated in the housing portion 110 (described in detail below) of the upper cartridge 100. Thereby, the medical instrument suite 1000 according to the present embodiment is obtained. Picking up of the upper cartridge 100 after the electronic device 300, the analyte sensor 400, and the puncturing member 500 is as shown in Fig. 6A and Fig. 6B. In some examples, as shown in Fig. 6A, a projection is performed along the radial direction of the medical instrument suite 1000 and the puncturing member 500 is covered by the upper cartridge 100. In this case, the puncturing member 500 is not exposed so that the user can be prevented from being mistakenly injured by the puncturing member 500 as much as possible.

Specifically, in some examples, as shown in Fig. 6B, the upper cartridge 100 may include a receiving portion 110, a moving main body 120, a first driving mechanism 131, a second driving mechanism 132, and a pressing portion 140.

In some examples, the moving main body 120 may be configured to move along a defined movement path. In some examples, the moving main body 120 may be releasably retained to the upper cartridge 100, and the moving main body 120 may be configured to move along a movement path defined by the upper cartridge 100 when released.

In some examples, the first driving mechanism 131 may be configured to apply action on the moving main body 120 in a manner directing toward the proximal end (the proximal end refers to one end close to the host) to move the moving main body 120 along the movement path defined by the upper cartridge 100.

In some examples, the pressing portion 140 may be pressed to enable the first driving mechanism 131 apply action on the moving main body 120.

In some examples, the receiving portion 110 may be configured to pick up and accommodate the analyte sensor 400 and the electronic device 300 (the analyte sensor 400 and the electronic device 300 after being assembled together), and to pick up and accommodate the analyte sensor 400 and the electronic device 300 to the receiving portion 110 when the upper cartridge 100 and the lower cartridge 200 are sealingly assembled.

In some examples, when picking up and accommodating the analyte sensor 400 and the electronic device 300, the receiving portion 110 may not destroy the sealing state of the accommodating chamber 210. In other words, after the upper cartridge 100 and the lower cartridge 200 are sealingly assembled, the sealing state of the accommodating chamber 210 may not be destroyed. Thereby, the sensing portion 410 of the analyte sensor 400 can be placed in the sealed accommodating chamber 210, reducing the contamination of the sensing portion 410 due to the environment outside the accommodating chamber 210.

In some examples, when the upper cartridge 100 and the lower cartridge 200 are disassembled, the sealing state of the accommodating chamber 210 may be destroyed at the same time.

In some examples, a user may disassemble the upper cartridge 100 and the lower cartridge 200 while using the medical instrument suite 1000, wherein the upper cartridge 100 is configured to apply the analyte sensor 400 and the electronic device 300 accommodated in the receiving portion 110 to a host. In this case, when the user needs to use the medical instrument suite 1000, the user can easily use the medical instrument suite, by disassembling the upper cartridge 100 from the lower cartridge 200 and then applying the picked-up analyte sensor 400 and the electronic device 300 to the host through the upper cartridge 100, without performing the assembly on his own.

In some examples, the receiving portion 110 may be provided on the moving main body 120; when the moving main body 120 moves, the receiving portion 110 may move. Thereby, the electronic device 300 and the analyte sensor 400 can be enabled to move along a defined path.

In some examples, when the moving main body 120 is released, the moving main body 120 may be driven by the driving mechanism 130 to a proximal end to push the electronic device 300, the analyte sensor 400, and the puncturing member 500 accommodated in the receiving portion 110 toward the host. In addition, analyte sensor 400 may be placed at least partially under the skin of a host by puncturing member 500.

In some examples, the upper cartridge 100 may include a clamping portion 121. In some examples, the clamping portion 121 may be configured to clamp the main body portion 510 of the puncturing member 500. Additionally, in some examples, the upper cartridge 100 may further include a second driving mechanism 132 configured to apply action on the clamping portion 121 in a manner directing toward the distal end (the distal end refers to one end away from the host). When the clamping portion 121 clamping the puncturing member 500 is released, the clamping portion 121 may be driven by the second driving mechanism 132 to the distal end to move the puncturing member 500 away from the host. In this case, the puncturing member 500 can be caused to leave the host after the puncturing (rebounding).

In some examples, when the medical instrument suite 1000 needs to be used, the upper cartridge 100 may be disassembled from the lower cartridge 200, i.e., the upper cartridge 100 is pulled out from the lower cartridge 200; at this time, the sensing portion 410 and the sharp object 520 are pulled out from the accommodating chamber 210; the electronic device 300, the analyte sensor 400, and the puncturing member 500 are accommodated in the pulled-out upper cartridge 100, and then by pressing the pressing portion 140, the upper cartridge 100 finally moves the electronic device 300, the analyte sensor 400, and the puncturing member 500 towards the host; the sensing portion 410 of the analyte sensor 400 is implanted under the skin of the host, the electronic device 300 is attached to the body surface of the host in a manner of being coupled with the connecting portion 420 of the analyte sensor 400, and the puncturing member 500 is pulled out after piercing into the host.

In some examples, the puncturing depth may be preconfigured through the upper cartridge 100 and the puncturing member 500 is enabled to be pulled out after the puncturing. Thereby, the upper cartridge 100 can be used for the purpose of quick puncture, painless puncture, and the like, thereby reducing the user's feeling of pain. In addition, the one-handed operation can be facilitated by performing puncturing through the upper cartridge 100.

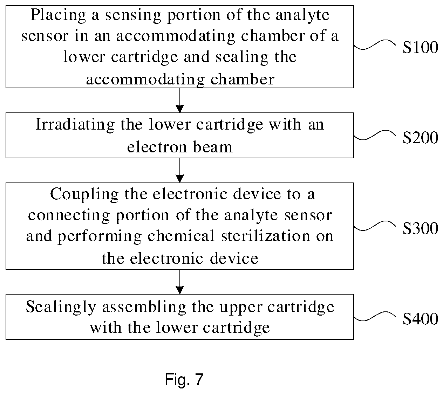

Fig. 7 is a flow chart showing a sterilization method for a medical instrument suite 1000 to which the present disclosure relates.

In some examples, as shown in Fig. 7, a sterilization method for a medical instrument suite 1000 may include: placing the sensing portion 410 of the analyte sensor 400 in the accommodating chamber 210 of the lower cartridge 200, sealing the accommodating chamber 210 (step S100), irradiating the lower cartridge 200 with an electron beam (step S200), coupling the electronic device 300 to a connecting portion 420 of the analyte sensor 400, performing chemical sterilization on the electronic device 300 (step S300), and sealingly assembling the upper cartridge 100 and the lower cartridge 200 (step S400).

In some examples, as described above, the sterilization method for the medical instrument suite 1000 may include: placing the sensing portion 410 of the analyte sensor 400 in the accommodating chamber 210 of the lower cartridge 200, and sealing the accommodating chamber 210 (step S100). In this case, the sensing portion 410 of the analyte sensor 400 may be sealed in the accommodating chamber 210 of the lower cartridge 200 such that the sensing portion 410 can be isolated from the external gas without subjecting the sensing portion 410 to the contamination due to the external gas environment.

In some examples, the accommodating chamber 210 may be sealed with a seal 220. In some examples, the seal 220 may be wax in a molten state. Thereby, the sealing of the accommodating chamber 210 can be achieved, and the seal 220 may be subsequently destroyed by mechanical force. For example, when the medical instrument suite 1000 is used, pulling out the analyte sensor 400 may destroy the sealing state.

In some examples, in step S100, the sharp object 520 of the puncturing member 500 may further be placed into the accommodating chamber 210 of the lower cartridge 200 and then the accommodating chamber 210 is sealed. In some examples, the entirety of the sharp object 520 may be placed in the accommodating chamber 210. In some examples, a portion of the sharp object 520 may be placed in the accommodating chamber 210. In this case, the sharp object 520 may be isolated from the external gas without causing the sharp object 520 to be contaminated by the external gas environment.

In some examples, in step S100, a desiccant may also be placed in the accommodating chamber 210 and then the accommodating chamber 210 is sealed. Thereby, the environment inside the accommodating chamber 210 can be brought into a dry state by the desiccant, so that the sensing portion 410 sealed inside the accommodating chamber 210 is not affected by the moisture in the environment.

In some examples, as described above, the sterilization method for the medical instrument suite 1000 may include: irradiating the lower cartridge 200 with an electron beam (step S200). In this case, the sensing portion 410 and/or the sharp object 520 sealed in the accommodating chamber 210 can be sterilized by using an electron beam.

In some examples, in step S200, the lower cartridge 200 may also be irradiated with gamma ray radiation or X-ray radiation.

In some examples, in step S200, the irradiation dose of the electron beam is greater than or equal to 15kGy and less than or equal to 50kGy. Thereby, it is able to further effectively kill microorganisms at the sensing portion 410 and/or the sharp object 520 sealed in the accommodating chamber 210.

In some examples, as described above, the sterilization method for the medical instrument suite 1000 may include: coupling the electronic device 300 to the connecting portion 420 of the analyte sensor 400, and performing chemical sterilization on the electronic device 300 (step S300). In this case, by firstly performing electron beam sterilization on the sensing portion 410 and/or the sharp object 520, then coupling the electronic device 300 to the connecting portion 420 of the analyte sensor 400, the function of the electronic device 300 can be protected from the influence of the electron beam.

In some examples, in step S300, the electronic device 300 may not be placed in the irradiation range of the electron beam. In some examples, the electronic device 300 may be coupled to the connecting portion 420 after the electron beam irradiation is finished.

In some examples, in step S300, the electronic device 300 may be chemically sterilized by placing the electronic device 300 in a chemical gas atmosphere. In some examples, both the lower cartridge 200 and the electronic device 300 may be placed in a chemical gas atmosphere after the electronic device 300 is coupled to the connecting portion 420. Thereby, the electronic device 300 can be sterilized by chemical gas.

In some examples, in step S300, the chemical gas may be ethylene oxide. In turn, the electronic device 300 can be chemically sterilized by ethylene oxide. In this case, since the sensing portion 410 and/or the sharp object 520 have been sealed in the accommodating chamber 210, the chemical gas will not enter the accommodating chamber 210, so that the chemical gas does not damage the composition or substances in the sensing portion 410.

In some examples, as described above, the sterilization method for the medical instrument suite 1000 may include: sealingly assembling the upper cartridge 100 and the lower cartridge 200 (step S400).

In some examples, in step S400, the electronic device 300 may be sealed between the upper cartridge 100 and the lower cartridge 200 by the sealing assembly of the upper cartridge 100 and the lower cartridge 200, thereby reducing the influence on the electronic device 300 from bacteria in the external gaseous environment.

In some examples, after step S400, the method may further include sealing packaging of the medical instrument suite 1000 with a sealing bag, whereby the gas tightness of the medical instrument suite 1000 can be further ensured.

The sterilization method for the medical instrument suite 1000 according to the present embodiment separately sterilize the analyte sensor 400 and the electronic device 300, electron beam is used for the analyte sensor 400 sterilization and chemical sterilization is used for the electronic device 300,enabling to protect the electronic device 300 from the interference of the electron beam sterilization and protect the electronic device 300 without requiring to use a structure such as an electron beam shielding member, resulting in simplification of the structure of the medical instrument suite 1000,. The sterilization of the analyte sensor 400, the puncturing member 500, and the electronic device 300 can thereby be facilitated. Meanwhile when the medical instrument suite 1000 is used, the analyte sensor 400 and the electronic device 300 can be applied to a host directly through the upper cartridge 100 without an additional assembly by the user, facilitating the use of user. The sterilization method for the medical instrument suite 1000 of the present embodiment enables the sensing portion 410 of the analyte sensor 400 and the sharp object 520 of the puncturing member 500 to be sealed in the accommodating chamber 210, and after the analyte sensor 400 and the puncturing member 500 are subjected to the electron beam sterilization, the sensing portion 410 and the sharp object 520 can be prevented from being contaminated by the gaseous environment outside the accommodating chamber 210; the electron beam can be prevented from affecting the function of the electronic device 300 by, after the electron beam sterilization, connecting the electronic device 300 to the analyte sensor 400.

Although the present disclosure has been specifically described above with reference to the accompanying drawings and embodiments, it will be understood that the above description does not limit the disclosure in any form. Those skilled in the art may make variations and changes to the disclosure as required without departing from the true spirit and scope of the disclosure, and these variations and changes fall within the scope of the disclosure.

## Claims

1. A sterilization method for a medical instrument suite, **characterized in that** the medical instrument suite includes an analyte sensor, an electronic device, a lower cartridge having an accommodating chamber, and an upper cartridge sealingly assembled with the lower cartridge, wherein the analyte sensor has a sensing portion capable of reacting with an analyte to generate a sensing signal, and a connecting portion which is electrically connected with the sensing portion; the electronic device is configured to be capable of being coupled with the connecting portion and receiving the sensing signal generated by the sensing portion,
the sterilization method includes:
placing the sensing portion of the analyte sensor in the accommodating chamber of the lower cartridge,
sealing the accommodating chamber in such a manner that the sensing portion is sealed,
irradiating the lower cartridge with an electron beam,
coupling the electronic device to the connecting portion, and
assembling sealingly the upper cartridge and the lower cartridge.

2. The sterilization method according to claim 1, **characterized in that**
an irradiation dose of the electron beam is greater than or equal to 15kGy and less than or equal to 50kGy.

3. The sterilization method according to claim 1, **characterized in that**
the medical instrument suite further includes a puncturing member, wherein the puncturing member includes a main body portion, and a sharp object provided on the main body portion and having an accommodating groove for accommodating the sensing portion, the sharp object being sealed in the accommodating chamber.

4. The sterilization method according to claim 3, **characterized in that**
a projection is performed along a radial direction of the instrument suite and the puncturing member is covered by the upper cartridge.

5. The sterilization method according to claim 1, **characterized by** further including placing the electronic device in a chemical gas atmosphere after coupling the electronic device to the connecting portion.

6. The sterilization method according to claim 5, **characterized in that**
the chemical gas is ethylene oxide.

7. The sterilization method according to claim 1, **characterized in that**
the accommodating chamber is sealed with wax in a molten state.

8. The sterilization method according to claim 1, **characterized in that**
the electronic device is not within an irradiation range of the electron beam.

9. The sterilization method according to claim 1, **characterized in that**
the lower cartridge further includes a desiccant provided in the accommodating chamber.

10. The sterilization method according to claim 1, **characterized in that**
an outer contour of the upper cartridge is matched with an inner contour of the lower cartridge, and an outer edge of the upper cartridge includes a rubber ring.
